# EUROPÄISCHE PATENTSCHRIFT

(11) **EP 1 396 261 B1**
(45) Veröffentlichungstag und Bekanntmachung des Hinweises auf die Patenterteilung: **27.10.2004**
(21) Anmeldenummer: 03016729.0
(22) Anmeldetag: 22.07.2003
(51) Int. Cl.: A61K 7/48, A61K 31/52

(54) **Coffein enthaltende Zusammensetzung als Hauptflegemittel**
Skincare composition containing caffeine
Composition pour les soins de la peau contenant de la caféine

(30) Priorität: 06.09.2002 DE 10241395
(43) Veröffentlichungstag der Anmeldung: 10.03.2004
(73) Patentinhaber: Alcina Cosmetic, Dr. Kurt Wolff GmbH & Co.KG, 33611 Bielefeld (DE)
(72) Erfinder: Bornemann, Petra, 33647 Bielefeld (DE); Brandner, Johanna, 22303 Hamburg (DE); Klenk, Adolf, 33415 Verl (DE); Menzel, Birgit, 32756 Detmold (DE); Tsianakas, Athanasios, 20251 Hamburg (DE)
(74) Vertreter: Grünecker, Kinkeldey, Stockmair & Schwanhäusser Anwaltssozietät

(56) Entgegenhaltungen:
- WO-A-00/69407
- DE-A- 10 133 203
- DE-A- 19 824 727
- DE-U- 29 916 868
- FR-A- 2 620 024
- GB-A- 2 177 918
- US-A- 5 922 331
- US-A1- 2002 150 597
- DATABASE WPI Week 200016 Derwent Publications Ltd., London, GB; AN 2000-176892 XP002236590 & JP 2000 026301 A (INAMURA KK)
- DATABASE WPI Week 199345 Derwent Publications Ltd., London, GB; AN 1993-358162 XP002236591 & SU 1 770 352 A (AEROZOL SCI PRODN ASSOC)

## Beschreibung

Die vorliegende Erfindung betrifft die Verwendung von Coffein als aktiven Bestandteil für Hautpflegemittel.

### Stand der Technik

Die Haut ist das größte Organ des Menschen. Unter ihren vielen Funktionen (beispielsweise zur Wärmeregulation und als Sinnesorgan) ist die Barrierefunktion sowie Schutzfunktion, die das Austrocknen der Haut (und damit letztlich des gesamten Organismus) verhindert, die wohl Wichtigste. Gleichzeitig wirkt die Haut als Schutzeinrichtung gegen das Eindringen und die Aufnahme von außen kommender Stoffe. Insbesondere sind hier schädliche UV-Strahlen zu nennen, mikrobiologische Angriffe und Penetration von hautfremden Schadstoffen. Bewirkt wird diese Barrierefunktion durch die Epidermis (Oberhaut), welche als äußerste Schicht die eigentliche Schutzhülle gegenüber der Umwelt bildet. Besondere Beachtung gilt der äußeren Homschicht (Stratum Corneum), einer widerstandsfähigen Zellschicht, deren einzelne Homzellen durch die sogenannte Lipid-Barriere verbunden sind. Die Lipid-Barriere schützt die Haut vor Austrocknung und hält den Quellungszustand aufrecht. Mit etwa einem Zehntel der Gesamtdicke der Haut ist die Epidermis gleichzeitig die dünnste Schicht der Haut. Diese umfasst, neben der Epidermis noch das darunter gelegene mesodermale Bindegewebe des Coriums (Lederhaut, Unterhaut). Beide Schichten sind durch ein System papillen- oder leistenförmiger Coriumsvorwölbungen und tief zwischen diese hineinragender Epidermiszapfen innig miteinander verzahnt. Vom reich durchbluteten Corium her folgt auch die Ernährung der Epidermis. Pigmentzellen im Corium liefern die Pigmente, die der Haut ihre Färbung verleihen und in den Coriumpapillen liegen die Sinnesrezeptoren der Hautsinne für Tastempfindung, Schmerzempfindung und Temperaturempfindung.

Die oberflächlichen Zelllagen der Epidermis unterliegen einem ständigen Verschleiß. Zusätzlich kann die Oberhaut sehr empfindlich mit Reizungen, Rötungen, Juckreiz, Schuppungen und Rissbildungen auf äußere Einflüsse reagieren. Um daher den Schutz optimal zu gewährleisten, erneuert sich die Epidermis deshalb innerhalb von ca. 30 Tagen vollständig. Diese Epidermiserneuerung erfolgt aus einer basalen, zeitlebens teilungsaktiven Keimschicht durch eine kontinuierliche Ersetzung.

Um die Hautbarriere der Epidermis zu verbessern, werden inzwischen viele kosmetische Zusammensetzungen angeboten, durch welche üblicherweise von außen Fettstoffe der Haut zugeführt werden. Solche Zusammensetzungen haben aber eine Reihe von Nachteilen. Die Penetration in die Haut ist oft eher gering, so dass das Hautgefühl unangenehm fettig sein kann. Meist sind die Fettstoffe auch körperfremd und können so eine geschwächte Hautbarriere nur unbefriedigend regenerieren. Gerade bei der männlichen Haut, die hormonbedingt eine hohe Talgdrüsenaktivität zeigt, wird das Problem der geeigneten Rückfettung der Haut besonders anschaulich. Die Haut wird rasch übersättigt und es entsteht ein unangenehmes Hautgefühl. Bei der männlichen Haut kommt erschwerend weiter hinzu, dass die hormonbedingte Beeinträchtigung der Haut nahezu konstant bis ins hohe Alter vorhanden ist, was durch die konventionellen Hautbehandlungsmittel nur unzureichend ausgeglichen werden kann. Die hormonbedingte Beeinträchtigung in der männlichen Haut führt dazu, dass insbesondere die Regenerationsleistung, d. h. die Erneuerung der Oberhaut gefährdet sein kann. Die Aktivität der Keratinozyten nimmt ab, d. h. die Zellteilung bzw. Hautregeneration und somit die Bildung der körpereigenen Hautbarriere nimmt ab.

Es besteht daher ein Bedarf für Zusammensetzungen, die eine aktive Komponente enthalten, die insbesondere bei der männlichen Haut die Erneuerung der Epidermis unterstützt und die hormonelle Beeinträchtigung ausgleicht.

Kosmetische und dermatologische Zubereitungen für die Pflege der Haut sind bekannt. So offenbart beispielsweise die DE-A-199 34 945 derartige Zusammensetzungen auf der Grundlage von O/W-Emulsionen, die im wesentlichen auf einer Mischung aus langkettigen Fettsäuren, Mono- und/oder Diglyceriden von Fettsäuren, ethoxylierten Fettsäureestern, unpolaren Lipiden, Fettalkoholen, lipophilen Konsistenzgebern, hydrierten Polyisobutenen und polaren Lipiden basieren. Dieses Dokument offenbart auch die Einführung von Wirkstoffen in die Zusammensetzungen, wie beispielsweise Coffein, wobei über den Effekt des Coffeins allerdings keine Aussagen gemacht werden.

Der Einsatz von Coffein in kosmetischen Zusammensetzungen ist darüber hinaus in einer ganzen Reihe von weiteren Druckschriften beschrieben. So offenbart die WO-A-02/05755 gelartige kosmetische Zusammensetzungen zur Fettreduktion, die als fettabbauende Komponente beispielsweise wasserfreies Coffein enthalten können. Der Einsatz von Coffein ist darüber hinaus auch für Zusammensetzungen bekannt, die bei Haarausfall zum Einsatz kommen sollen. So offenbart beispielsweise die DE-A-197 01 651 Biotin enthaltende liposomale Formulierungen, die als zusätzlichen Wirkstoff auch Coffein enthalten. Die EP-A-1 179 334 beschreibt ein Haartonikum zur Vorbeugung oder Behandlung von Haarausfall, das ebenfalls Coffein als Wirkstoff enthalten kann.

Weitere kosmetische Zusammensetzungen, die Coffein enthalten können, sind auch aus den europäischen Anmeldungen EP-A-665 001 und EP-A-692 241 bekannt.

Die FR-A-2,620,024 offenbart, dass Coffein als Radikalfänger eingesetzt werden kann, insbesondere bei Sonneneinstrahlung.

Die US 2002/150597 offenbart bestimmte Zusammensetzungen, die zur Behandlung von Falten dienen können.

Die US-A-5,922,331 offenbart Zusammensetzungen, die zur Hautglättung eingesetzt werden können.

Die DE 198 24 727 A und die DE 101 33 203 A betreffen Zusammensetzungen, die trockene Hautzustände behandeln oder hypoaktive Hautzustände.

Die DE 299 16 868 U offenbart Zusammensetzungen zur Behandlung von Erschöpfung, Müdigkeit oder Durchblutungsstörungen.

Die SU 1 770 352 A offenbart Zusammensetzungen, die der Hautaustrocknung entgegenwirken sowie anti-allergische Reaktionen zeigen.

Diese Druckschriften offenbaren alle den Einsatz von Coffein in kosmetischen Zusammensetzungen, wobei das Coffein entweder als fettreduzierendes Mittel oder als Mittel gegen Haarausfall eingesetzt wird. Weitere Wirkungen des Coffeins sind in diesen Druckschriften nicht offenbart.

Die WO 00/69407 und die GB-A-2,177,918 offenbaren einen Einsatz von Zusaimmensetzungen zur Wundheilung, wobei derartige Zusammensetzungen Coffein enthalten können.

Die JP-2000-026301 A offenbart entzündungshemmende Zusammensetzungen.

Pflegeprodukte für die männliche Haut sind inzwischen schon weit verbreitet erhältlich. Diese enthalten als aktive Komponenten beispielsweise Vitamin E.

Ausgehend vom oben geschilderten Stand der Technik stellte sich die vorliegende Erfindung die Aufgabe ein aktives Mittel aufzufinden, das unterstützend auf die Regeneration der Epidermis wirkt, für die Regeneration der männlichen Haut, die durch den Einfluss von männlichen Hormonen gestort sein kann bzw. für weibliche Haut mit erhöhtem Testosteronspiegel.. Dabei sollte das aktive Mittel bevorzugt in einfacher Art und Weise anwendbar sein, wobei insbesondere eine topische Anwendung erfolgen sollte, da dadurch eine einfachere Verabreichung sicher gestellt werden kann, im Vergleich beispielsweise mit einer systemischen Applikation. Gleichzeitig sollte das aktive Mittel einfach herstellbar und gut verarbeitbar sein, so dass es in üblichen kosmetischen und dermatologischen oder systemischen Formulierungen eingesetzt werden kann.

### Beschreibung der Erfindung

Überraschend wurde gefunden, dass Coffein eine Verbindung ist, die die oben aufgelisteten Aufgaben löst. Der Einsatz von Coffein unterstützt die Regenerierung der Epidermis, insbesondere auch bei der männlichen Haut, in der die Regeneration durch den Einfluss von männlichen Hormonen beeinträchtigt sein kann. Es wurde überraschend gefunden, dass Coffein die Schutzbarriere der männlichen Haut praktisch auf den ursprünglichen Zustand zurückführt, der vor dem Einfluss der männlichen Hormone geherrscht hat, so dass quasi ein juveniler Hautzustand wieder erreicht werden kann. Darüber hinaus wurde gefunden, dass auch die Aktivität der Keratinozyten durch Coffein deutlich gesteigert wurde.

### Kurze Beschreibung der Figuren

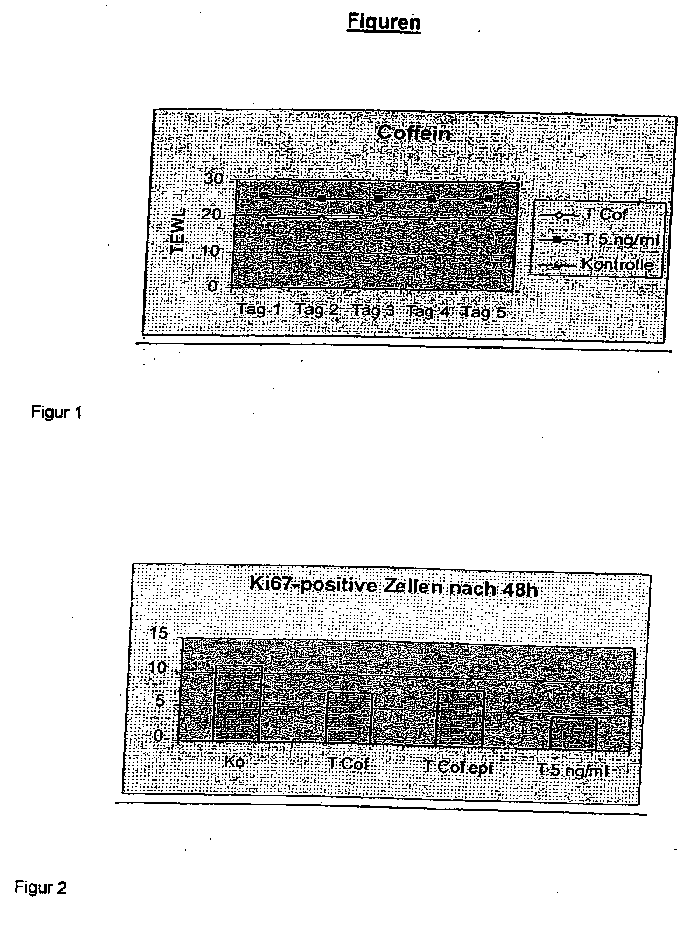

Figur 1 zeigt den Verlauf des epidermalen Wasserverlustes (TEWL), d. h. die Barriereschädigung im Normalzustand [Δ]. Bedingt durch die Präparation ist schon eine geringfügige Schädigung sichtbar. Unter dem Einfluß von 5 ng Testosteron (unter physiologische Gerumkonzentration) wird der Wasserverlust erhöht [□]. Der Versuch unter Zusatz von Coffein dagegen zeigt überraschender Weise, dass der TEWL wieder auf den Ausgangszustand zurück gedrängt werden konnte [0].

Figur 2 zeigt dagegen die Zellaktivität von Keratinozyten im Ausgangszustand [ko], bei Zusatz von Testosteron [T Sng/ml] sowie beim Zusatz von Coffein [T Cof und T Cof epi]. Diese Versuche wurden unter Einsatz spezieller Färbetechniken durchgeführt, so dass teilungsaktive Keratinozyten sichtbar gemacht und ausgezählt werden konnten. Wiederum zeigt sich, das Testosteron nachteilig wirkte, da diese Substanz die Teilungsaktivität der Keratinozyten herabsetzt. Durch Coffein wird dagegen diese Blockade der Zellaktivität weitgehend wieder aufgehoben.

Ein ganz besonders überraschender Effekt ist, dass die epicutane Applikation von Coffein bei dieser Versuchsanordnung zu besseren Resultaten führt, als die systemische Applikation. Coffein zeigt daher bei epicutaner Anwendung eine gute Penetration, so dass eine sehr zufrieden stellende Wirkung erreicht werden kann.

### Detaillierte Beschreibung der Erfindung

Die oben aufgelisteten guten Eigenschaften des Coffeins im Hinblick auf die Regeneration der Epidermis der männlichen Epidermis ermöglichen den Einsatz des Coffeins in kosmetischen und dermatologischen Zusammensetzungen zur Unterstützung dieser Regeneration. Das aufgefundene Eigenschaftsprofil des Coffeins ist überraschend, da bislang lediglich die haarwuchsfördernde Aktivität sowie die fettreduzierende Aktivität des Coffeins bekannt waren. Die gezeigte Aktivität bei epicutaner Anwendung ermöglicht darüber hinaus den topischen Einsatz der Coffein enthaltenden Zusammensetzungen, was deren Einsatz vereinfacht, Nebenwirkungen, die beispielsweise bei systemischer Applikation auftreten können, unterdrückt und die erfindungsgemäße Verwendung im täglichen Leben ermöglicht, da die Coffein enthaltenden Formulierungen in der Form von Produkten des täglichen Lebens bereit gestellt werden können, wie als Shampoo, als Creme usw..

Die vorliegende Erfindung stellt daher die Verwendung nach Anspruch 1 zur Verfügung. Bevorzugte Aüsführungsformen dieser Verwendung sind in den Unteransprüchen angegeben.

Erfindungswesentlich ist in allen Fällen die Verwendung von Coffein als aktiver Bestandteil zur Unterstützung der Regeneration der Epidermis, der männlichen Epidermis. Insoweit betrifft die vorliegende Erfindung die Verwendung von Coffein zur Prophylaxe oder zur Therapie von Störungen, die durch die mangelnde Regeneration der Epidermis hervorgerufen werden.

Die Verwendung von Coffein kann in allen üblichen Zusammensetzungen erfolgen, die dem Fachmann bekannt sind, wie als Hautschutzcreme, Reinigungsmittel, Sonnenschutzlotion, Nährcreme, Tages- oder Nachtcreme, Körperlotion, desodorierende Zusammensetzungen, Duschgel, Gesichtsmaske, Rasiercreme, Gesichtswasser, After Shave Balsam usw.. In solchen Zusammensetzungen kann das Coffein in einer Menge von 0,001 bis 30 Gew.-% eingesetzt werden, vorzugsweise in einem Bereich von 0,05 bis 10 Gew.-% und insbesondere vorzugsweise 0,01 bis 2 Gew.-%.

Die erfindungsgemäße Verwendung betrifft pharmazeutische Zusammensetzungen, die zur Regenerierung von beeinträchtigter Haut eingesetzt werden, wobei diese männliche Haut oder weibliche Haut mit erhöhtem Testosteronspiegel ist.

Die erfindungsgemäße Verwendung des Coffeins kann bevorzugt in Kombination mit weiteren aktiven Substanzen erfolgen, wie Vitamine und Flavonoide, Lichtschutzfiltern, feuchtigkeitsspendenden Wirkstoffen oder anderen aktiven Bestandteilen. Zusätzlich können die bei der erfindungsgemäßen Verwendung hergestellten Zusammensetzungen übliche Bestandteile enthalten, wie sie im Folgenden dargelegt sind.

Die erfindungsgemäß Verwendung ermöglicht die Behandlung von Haut, insbesondere von männlicher Haut oder weiblicher Haut unter bestimmten Stoffwechselumständen wie bei erhöhtem Testosteronspiegel, zur Regenerierung der Epidermis, wobei diese Behandlung den Schritt der Auftragung einer Coffein enthaltenden Formulierung auf die Haut umfasst. Die Auftragung kann entweder auf den Hautflächen stattfinden, die gezielt behandelt werden sollen oder die Auftragung erfolgt auf benachbarten oder anderen Hautflächen, je nach eingesetzter Formulierung.

Die erfindungsgemäße Verwendung kann in der Form kosmetischer und pharmazeutischer Formulierungen erfolgen, die Coffein als wesentlichen Bestandteil enthalten. Diese Formulierungen dienen der Behandlung von Haut zur Unterstützung der Regenerierung der Epidermis, insbesondere zur Verwendung auf der männlichen Haut oder auf der weiblichen Haut bei bestimmten Stoffwechselumständen, insbesondere bei erhöhtem Testosteronspiegel. Bevorzugte Zusammensetzungen in Übereinstimmung mit der vorliegenden Erfindung ergeben sich aus den vorstehenden und nachfolgenden Ausführungen sowie den explizit aufgeführten Beispielen.

Die erfindungsgemäß verwendeten Zusammensetzungen können durch den Fachmann bekannte Herstellungsverfahren hergestellt werden. Die dazu notwendigen Vorrichtungen und Prozeßparameter sind dem Fachmann bekannt. So kann beispielsweise Coffein oder eine Coffein enthaltende Zubereitung mit einer Basiszusammensetzung für gewünschte Formulierungen (Creme, Shampoo etc.) vermischt und so die erfindungsgemäße Formulierung erhalten werden. Da Coffein relativ gut gehandhabt werden kann, gibt es im Hinblick auf das erfindungsgemäße Herstellungsverfahren keine besonderen Beschränkungen.

In der fertigen kosmetischen Formulierung kann Coffein mit allen bekannten und üblichen Hilfsstoffen, Emulgatoren, Stabilisatoren, Konservierungsstoffen und/oder Lichtschutzfilter kombiniert werden. Ebenso vorstellbar sind weitere biologisch wirksame Stoffe oder Zubereitungen, wie z.B. Aminosäuren, Oligo- oder Polypeptide, natürlicher oder biotechnologischer Herkunft, Vitamine, Saccharide, Pflanzenextrakte sowie deren gereinigte Fraktionen, enthaltend Polyphenole, Flavonoide, Terpenoide und andere sekundäre Pflanzenbegleitstoffe.

Nachfolgende Aufzählung einzelner Stoffe ist beispielhaft, benennt lediglich bevorzugte Ausführungsformen und erhebt keinen Anspruch auf Vollständigkeit. Sie soll den Stoffcharakter der möglichen Kombinationen zeigen, die sich dem Produktentwickler eröffnen.

Reinigungsprodukte, die als Gel, Aerosol- oder Non Aerosolschaum appliziert werden, setzen sich vorzugsweise aus einer Kombination anionischer Tenside, wie Laurylethersulfat, Laurylsulfat, Ethercarboxylate mit variablem Ethoxylierungsgrad, Ethersulfonate auf der Grundlage von Aminosäuren, Zuckern und Fettalkoholen zusammen. Diese werden mit amphoteren Tensiden wie Cocamidopropyl Betain, Tauraten kombiniert. Zur Verbesserung des Hautgefühls und der Rückfettung können nichtionische Tenside auf Zucker und Lipidbasis eingesetzt werden.

Weitere Stoffbeispiele für die jeweiligen erfindungsgemäßen Produktgrundlagen sind:
Acylglutamate, z.B. Natrium Acylglutamat, Na-Laurylglutamat, Na-Capryl/Capringlutamat, DI-TEA-palmitoylaspartat, Acylpeptide wie Palmitoyl-hydrolysiertes Milchprotein, Sojaprotein, Collagen oder Keratin. Cocosfettsäurekondensate der genannten Proteinquellen. Sarcosinate, z.B. Myristoylsarcosin, TEA-Lauroylsarcosin, Na-Lauroylsarcosin, Na-Cocoylsarcosin, Taurate der Fettsäuren mit einer Kettenlänge von C10 bis C20, gesättigt oder ungesättigt, Acyllysinate, -alaninate oder -glycinate des zuvor beschriebenen Fettsäurespektrums.

Co-Tenside wie Cocamidopropyl Betaine, Decyl Polyglycoside, Dodecyl Polyglycoside, Disodium Laureth Sulfosuccinate, Trilaureth 4-Phosphate, Di/Mono Sodium Cocoamphoacetate.

Als Co-Emulgatoren oder Rückfetter kommen in Frage:
PEG-nn hydriertes Ricinusöl, PEG-6 Caprylsäure/Caprinsäureglyceride, Glyceryloleat im Gemisch mit Propylenglycol, PEG-9-Stearat, Ceteth-2, Ceteth-20, Polysorbat 60, Glycerylstearat im Gemisch mit PEG100, Glycerylmyristat, Glyceryllaurat, PEG-40-Sorbitanperoleat, Laureth-4, Ceteareth-3, Isostearylglycerylether, Cetylstearylalkohol im Gemisch mit Natrium Cetylstearylsulfat, Lameth-23, Steareth-2, Glycerylstearat im Gemisch mit PEG-3() Stearat. PEG-40-Stearat, Glycol Distearat, PEG-22-Dodecyl Glycol, Polyglyceryl-2 PEG-4-Stearat, Ceteareth-20, Methylglucosesesquistearat, Steareth-10, PEG-20-Stearat, Steareth-2 im Gemisch mit PEG-8 Distearat, Steareth-21, Steareth-20, Isosteareth-20, PEG-45 Dodecyglycol-Copolymer, Methoxy-PEG-22 Dodecylglycol-Copolymer, PEG-40-Sorbitanpero1eat, PEG-40-Sorbitanperisostearat, PEG-20-Glycerylstearat, PEG-20-Glycerylstearat, PEG-8- Bienenwachs, Polyglyceryl-2-laurat, I-sostearyldiglycerylsuccinat, Stearamidopropyl- PG-dimoniumchloridphosphat, Glycerylstearat SE, PEG-20-Methylglucosesesquistearat, Ceteareth-12, Glycerylstearatcitrat, Cetylphosphat, Sorbitansesquioleat, Trilaureth-4-Phosphat, Polyglycerylglucosedistearat, Kaliumcetylphosphat, Isosteareth-10, Polyglyceryl-2-sesquiisostearat, Ceteth-10, Oleth-20, Isoceteth-20, Glycerylsterat im Gemisch mit Ceteareth-20, Ceteareth-12, Cetylstearylalkohol und Cetylpalmitat, Cetylstearylalkohol im Gemisch mit PEG-20.Stearat, PEG-30-Stearat, PEG-40-Stearat, PEG-100-Stearat.

Es ist dem Fachmann natürlich bekannt, daß anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel. Stabilisatoren, Alkohol, Wasser, Salze, antimikrobiell, proteolytisch oder keratolytisch wirksame Substanzen usw.
Entsprechend können kosmetische oder topische dermatologische Zusammensetzungen im Sinne der vorliegenden Erfindung, je nach ihrem Aufbau, beispielsweise verwendet werden als Hautschutzcreme, Reinigungsmilch, Sonnenschutzlotion, Nährcreme, Tages- oder Nachtcreme usw. Es ist gegebenenfalls möglich und vorteilhaft, die erfindungsgemäßen Zusammensetzungen als Grundlage für pharmazeutische Formulierungen zu verwenden.

Günstig sind auch solche kosmetischen und dermatologischen Zubereitungen, die in der Form eines Sonnenschutzmittels vorliegen. Vorzugsweise enthalten diese neben dem findungsgemäß verwendeten Wirkstoff zusätzlich mindestens eine UVA-Filtersubstanz und/oder mindestens eine UVB-Filtersubstanz und/oder mindestens ein anorganisches Pigment.

Es ist aber auch vorteilhaft im Sinne der vorliegenden Erfindung, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden beispielsweise in Tagescremes gewöhnlich UV-A- bzw. UV-B-Filtersubstanzen eingearbeitet.

Vorteilhaft können erfindungsgemäße Zubereitungen Substanzen enthalten, die UV-Strahlung im UVB- Bereich absorbieren, wobei die Gesamtmenge der Filtersubstanzen z. B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen beträgt.

Die UVB-Filter können öllöslich oder wasserlöslich sein. Als Öllösliche Substanzen sind z. B. zu nennen:
- 3-Benzylidencampher und dessen Derivate, z. B. 3-(4-Methylbenzyliden)campher,
- 4-Aminobenzoesäure Derivate, vorzugsweise 4-(Dimethylamino)-benzoesäure(2-ethylhexyl)ester, 4-(Dimethylamino )benzoesäureamylester;
- Ester der Zimtsäure, vorzugsweise 4-Methoxyzimtsäure(2-ethylhexy1)ester, 4-Metboxyzimtsäureisopentylester; - Ester der Salicylsäure, vorzugsweise Salicylsäure(2-ethylhexyl)ester, Salicylsäure.(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'- Dihydroxy-4-metboxybenzophenon;
- Ester der Benzalmalonsäure, vorzugsweise 4-Methoxybenzalmalonsäuredi(2-ethylhexyl)ester;- 2,4,6- Trianilino-(p-carbo-2'-ethyl-1'-hexyloxy)-1,3,5-triazin.

Als wasserlösliche Substanzen sind vorteilhaft:
2-Phenylbenzimidazol-5-sulfonsäure und deren Salze, z. B. Natrium-, Kalium- oder Triethanolammonium-Salze, - Sulfonsäure-Derivate von Benzophenonen, vorzugsweise 2-Hydroxy-4-methoxybenzophenon-5-sulfonsäure und ihre Salze;
Sulfonsaure-Derivate des 3-Benzylidencamphers, wie z. B. 4-(∼Oxo-3-bornylidenmethyl)benzolsulfonsäure, 2-Methyl-5-(2-oxo-3-bornylidenmethyl)sulfonsäure und ihre Salze.

Die Liste der genannten UVB-Filter, die erfindungsgemäß Verwendung finden können, ist nicht limitierend aufzufassen.

Es kann auch von Vorteil sein, in erfindungsgemäßen Zubereitungen UVA-Filter einzusetzen, die üblicherweise in kosmetischen und/oder dermatologischen Zubereitungen enthalten sind. Bei solchen Filtersubstanzen handelt es sich vorzugsweise um Derivate des Dibenzoylmethans, insbesondere um 1-(4'-tert.Butylphenyl)-3-(4'-methoxyphenyl)-propan-1,3-dion und um I-Phenyl-3-(4.-isopropylphenyl)propan-1,3-dion. Auch Zubereitungen, die diese Kombinationen enthalten, sind Gegenstand der Erfindung. Es können die gleichen Mengen an UVA-Filtersubstanzen verwendet werden, welche für UVB-Filtersubstanzen genannt wurden.

Kosmetische und/oder dermatologische Zubemitungen im Sinne der vorliegenden Erfindung können auch anorganische Pigmente enthalten, die üblicherweise in der Kosmetik zun Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon sowie Abwandlungen, bei denen die Oxide die aktiven Agentien sind. Besonders bevorzugt handelt es sich um Pigmente auf der Basis von Titandioxid. Es können die für die vorstehenden Kombinationen genannten Mengen verwendet werden.

Die erfindungsgemäß verwendeten kosmetischen und dermatologischen Zubereitungen können kosmetische Wirk-, Hilfs- und/ oder Zusatzstoffe enthalten, wie sie üblicherweise in solchen Zubereitungen verwendet. werden, z. B. Antioxidationsmittel, Konservierungsmittel, Bakterizide, Parfüme, Substanzen zum Verbinden des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polyglycole, Schaumstabilisatoren, Elektrolyte, Organische Lösungsmittel oder Silikonderivate.

Es ist vorteilhaft, den Zubereitungen im Sinne der vorliegenden Erfindung weitere antirritative oder antientzündliche Wirkstoffe zuzugeben, insbesondere Batylalkohol (α-Octadecylglyccrylether), Selachylalkohol, Chimylalkohol (α-Hexadecylglycerylether), Bisabolol und/oder Panthenol.

Es ist ebenfalls vorteilhaft, den Zubereitungen im Sinne der vorliegenden Erfindung übliche Antioxidantien zuzufügen. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend der Aminosäuren (z. B. Glycin, Histidin, Tyrosin, Tryptophan) und deren Derivate-, Imidazole (z. B. Urocaninsäure ) und deren Derivate, Peptide wie DL-Carnosin, D-Carnosin, L-Carnosin und deren Derivate (z. B. Anserin), (Carotinoide, Carotine (z. B. a-Carotin, β-Carotin, Lycopin) und deren Derivate, (Chlorogensäure und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, Linoleylester sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfuximin) in sehr geringen verträglichen Dosierungen (z. B. pmol bis j.Qnol/kg), ferner (Metall)-Chelatoren (z. B. a-Hydroxyfettsäuren, Palmitinsäure, Phytinsäure, Lactoferrin), a-Hydroxysäuren (z. B. Citronensäure, Milchsäure, Apfelsäure, Fumarsäure), Huminsäuren, Gallensäure, Gallenextrakte, Bilirubin, Biliverdin, EDTA, EGTA und deren Derivate, ungesättigte Fettsäuren und deren Derivate (z. B. gamma-Linolensäure, Linolsäure, Ölsäure), Folsäure und deren Derivate, Furfurylidensorbitol und dessen Derivate, Ubichinon und Ubichinol und deren Derivate-, Vitamin C und Derivate (z. B. Ascorbylpalmitat, Mg-Ascorbylphosphat, Ascorbylacetat), Tocopherole und Derivate (z. B. Vitamin-E-acetat), Vitamin A und Derivate (Vitamin-A-palmitat oder -acetat) sowie Koniferylbenzoat des Benzoeharzes, Niacin und Niacinamid, Vitamine der B-Gruppe, Biotin, Rutinsäure und deren Derivate, a-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Hamsäure und deren Derivate, Mannose und deren Derivate, Zink. und dessen Derivate (z. B. ZnO, ZnSO4, organische Komplexe/Salze z.B. Zn-PCA, Zn-Lactat, Zn-Tartrat ) Selen und dessen Derivate (z. B. Selenmethionin), Stilbene und deren Derivate (z. B. Stilbenoxid, TransStilbenoxid) und die erfindungsgemäß geeigneten Derivate (Salze, Ester, Ether, Zucker, Nukleotide, Nuklcoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05-20 Gew.-%, insbesondere 1-10 Gew.-%, bezogen auf das Gesamtgewicht der Zubereitung.

Sofern Vitamin E und/oder dessen Derivate das oder die Anlioxidantien darstellen, ist es vorteilhaft, deren jeweilige Konzentration aus dem Bereich von 0,001-10 Gew.-%, bezogen auf das Gesamtgewicht der Formulierung, zu wählen. Zubereitungen gemäß der vorliegenden Erfindung können auch Verwendung als Grundlage für kosmetische oder dermatologische Desodorantien bzw. Antitranspirantien finden. Alle für Desodorantien bzw. Antitranspirantien gängigen Wirkstoffe können vorteilhaft genutzt werden, beispielsweise Geruchsüberdecker wie die gängigen Parfümbestandteile, Geruchsabsorber. beispielsweise die in der Patentoffenlegungsscbrift DE-P 4009 347 beschriebenen Schichtsilikate, von diesen insbesondere Montmorillonit, Kaolinit, Ilit, Beidellit, Nontronit, Saponit, Hectorit, Bentonit, Smectit, ferner beispielsweise Zinksalze der Ricinolsäuren.

Keimhemmende Mittel sind ebenfalls geeignet, in die Zubereitungen eingearbeitet zu werden. Vorteilhafte Substanzen sind zum Beispiel 2,4,4'-Trichlor-2' hydroxydiphenylether (Irgasan), 1,6-Di-(4-chlorphenylbiguanido )-hexan (Chlorhexidin), 3,4,4'- 'Trichlorcarbanilid, quaternierte Ammoniumverbindungen, Nelkenöl, Minzöl, Thymianöl, Triethylcitrat, Famesol (3,7,11-Trimethyl-2,6,10-dodecatrien-I-ol) sowie die in den Patentoffenlegungsschriften DB-3740 186, DE-39 38140, DE-42 04 321, DE-4229 707, DE-4309 372, DB-4411664, DE-195 41967, DE. 19543695, DE-19543 696, DE-19547160, DE-19602108, DE-19602110, DB-19602111, DE.19631 003, DE. 19631004 und DE-196 34 019 und den Patcntschriftcn DB42 29737, DE-42 37081, DB43 24 219, DE-44 29467, DE-44 23 410 und DE-195 16705 beschriebenen Wirkstoffe bzw. Wirkstoffkombinationen. Auch Natriumhydrogencarbonat ist vorteilhaft zu verwenden.

Die Menge solcher Wirkstoffe (eine oder mehrere Verbindungen) in den Zubereitungen betragt vorzugsweise 0,001 bis 30 Gew.-%, besonders bevorzugt 0,05-20 Gew.-%, insbesondere 1-10 Gew.-%, bezogen auf das Gesamtgewicht der Zubereitung.

Die Wasserphase der kosmetischen Zubereitungen kann auch Gelcharakter aufweisen, die neben einem wirksamen Gehalt an erfindungsgemäß eingesetzten Substanzen und dafür üblicherweise verwendeten Lösungsmitteln, bevorzugt Wasser, noch weitere organische Verdickungsmittel, z. B. Gummiarabicum, Xanthangummi, Natriumalginat, Stärke und Stärkederivate (z. B. Distärkephosphat), Cellulose, Cellulose-Derivate, vorzugsweise Methylcellulose, Hydroxymethylcelulose, Hydroxyethylcellulose, Hydroxypropylcellulose. Hydroxypropylmethylcellulose oder anorganische Verdickungmittel, z. B. Aluminiumsilikate, wie beispielsweise organisch modifizierte oder auch unmodifizierte Hectorite, Bentonite, oder dergleichen, oder ein Gemisch aus Polyethylenglykol und Polyethylenglykolstearat oder -distearat, enthalten. Das Verdickungsmittel ist in dem Gel z. B. in einer Menge zwischen 0, 1 und 30 Gew.-%, bevorzugt zwischen 0,5 und 15 Gew.-%, enthalten.

Ferner kann es von Vorteil sein. Zubereitungen grenz- bzw. oberflächenaktive Agentien zuzufügen, beispielsweise kationische Emulgatoren wie insbesondere quatemäre Tenside.

Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind Alkylbetain, Alkylamidopropylbetain und Alkyl-amidopropylhydroxysulfain. Die erfindungsgemäß verwendeten kationischen Tenside können femer bevorzugt gewählt werden aus der Gruppe der quaternären Ammoniumverbindungen, insbesondere Benzyltrialkylammoniumchtoride oder -bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder - bromide, Dialkyldimethylammoniumchloride oder -bromide, Alkylamidethyltrimethylammoniumethersulfate, Alkylpyridiniumsalze, beispielsweise Lauryl- oder Cetylpyrimidinium Chlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

Vorteilhaft sind auch, kationische Polymere (z. B. Jaguare C162 [Hydroxypropyl Guar Hydroxypropyltrimonium Chloride] bzw. modifizierte Magnesiumaluminiumsilikaten (z. B. Quatemium-18- Hectorit, welches z. B. unter der Handelsbezeichnung Bentone 38 bei der Firma Rheox erhältlich ist, oder Stearalkonium Hectorit, welches z. B. unter der Handelsbezeichnung Softisane Gel bei der Hüls AG erhältlich ist), einzusetzen.

Erfindungsgemäß verwendete Zubereitungen können vorteilhaft auch Ölverdickungsmittel enthalten, um die taktilen Eigenschaften der Emulsion zu verbessern. Vorteihafte Ölverdickungsmittel im Sinne der vorliegenden Erfiindung sind beispielsweise weitere Feststoffe, wie z. B. hydrophobe Siliciumoxide des Typs Aerosil., welche von der Degussa AG erhält1ich sind. Vorteilhafte Aerosiltypen sind beispielsweise Aerosil *OX50,* Aerosil 130, Aerosil 150, Aerosil 200, Aerosil 300, Aerosil 380, Aerosil MOX 80. Aerosil MOX 170, Aerosil COK 84, Aerosil R 202, Aerosil R 805, Aerosil R 812, Aerosil R 972, Aerosil R 974 und/oder Aerosil R976.

Ferner sind auch sogenannte Metallseifen (d. h. die Salze höherer Fettsäuren mit Ausnahme der Alkalisalze) vorteilhafte Verdickungsmittel im Sinne der vorliegenden Erfindung, wie beispielsweise Aluminium-Stearat, Zink-Stearat und/oder Magnesium-Stearat.

Ebenfalls vorteilhaft ist, Zubereitungen amphotere bzw. zwitterionische Tenside (z. B. Cocoamidopylbetain) und Moisturizer (z. B. Betain) zuzusetzen. Vorteilhaft zu verwendende amphotere Tenside sind beispielsweise Acyl-/dialkylethylendiamin, beispielsweise Natriumacylamphoacetal, Dinatriumacylamphodipropionat, Dinatriumalkylamphodiacetat, Natriumacylamphohydroxypropylsulfonat, Dinatriumacylamphodiacetat und Natriumacylamphopropionat, N-Alkylaminosäuuren, beispielsweise Aminopropylalkylglutamid, Alkylaminopropionsäure, Natriumalkylimidodipropionat und Lauroamphocarboxyglycinat.

Möglich sind alle Zubereitungsformen, insbesondere Pflegeemulsionen als W/O, O/W oder auch Mehrfachemulsionen des Typs W/O/W, alkoholisch-wässrige Lösung, zB. Lotions oder Kuren und Gesichtspackungen, Hautschutzcremes, Reinigungsmilch, Sonnenschutzlotion, Nährcremes, Tages- oder Nachtcremes usw.. Auch Produkte der Körperpflege wie Duschgele oder Körperlotions und Deodorantien bzw. Antitranspirantien sind geeignet.

Die vorliegende Erfindung wird durch die nachfolgenden Beispiele an Zusammensetzungen illustriert.

### FORMULIERUNGSBEISPIELE

| **Beispiel 1** | **- After Shave Balsam -** | g/100g |
|---|---|---|
| | Glycerylstearat | 1,00 |
| | Cetylpalmitat | 0,40 |
| | Dicaprylylether | 5,50 |
| | Hexylessigsäure-/n-Decausäure-Triglycerid Glycerin | 5,00 |
| | Bisabolol | 0,20 |
| | Coffein | 0,20 |
| | Carbomer | 0,20 |
| | NaOH (1 %) | 4,20 |
| | Haltbarmacher | n.B. |
| | Aqua | ad 100,00 |

| **Beispiel 2** | **- Gesichtswasser, pH Wert 5.**5 - | |
|---|---|---|
| | Ethanol | 10,00 |
| | Glycerin | 5,00 |
| | Propylenglycol | 1,50 |
| | hydrolysiertes Gluten aus Weizen | 0,30 |
| | Harnstoff | 0,50 |
| | Coffein | 0,30 |
| | Allantoin | 0,10 |
| | lösliches Collagen | 1,00 |
| | Parfum | 0,30 |
| | PEG-40 hydriertes Castoröl | 0,80 |
| | Haltbarmacher | n. B. |
| | Milchsäure | n.B. |
| | Aqua | ad 100 |

| **Beispiel 3** | - **Rasiercreme** - | |
|---|---|---|
| | Kokossäure | 10,00 |
| | Palmitinsäure | 10,00 |
| | Stearinsäure | 8,00 |
| | Glycerin | 9,00 |
| | Coffein | 0,4 |
| | Kaliumhydroxid | 7,0 |
| | Natriumhydroxid | 0,9 |
| | Parfum | 0,8 |
| | Aqua | ad 100 |

| **Beispiel 4** | **- Schützende Tagescreme -** | |
|---|---|---|
| | PEG-40 hydriertes Castoröl | 6,00 |
| | Cetearylalkohol | 0,30 |
| | Cera Alba | 0,70 |
| | Myristylmyristat | 1,00 |
| | Hexyldecanol | 5,00 |
| | Dicaprylylether | 4,00 |
| | Dioctylcyclohexan | 3,00 |
| | Vitis Vinifera | 0,50 |
| | Tocopherylacetat | 1,00 |
| | Octylmethoxycinnamat | 2,00 |
| | 4-Isobutyl-Dibenzoylmethan | 1,00 |
| | Ascorbylpalmitat | 0,20 |
| | Retinylpalmitat | 0,05 |
| | 1,4 Butylenglycol | 4,00 |
| | Carbomer | 0,30 |
| | Hexyldecanol (und) Hexyldecyllaurat | 1,00 |
| | KOH | 0,60 |
| | Coffein | 0,20 |
| | Parfum | 0,20 |
| | Haltbarmacher | n.B. |
| | Aqua | ad 100 |

| **Beispiel 5** | **- Tagesfluid -** | |
|---|---|---|
| | Natrium-Dihydroxycetylphosphat | 4,00 |
| | Hexyldecanol | 3,00 |
| | Parfum | 0,30 |
| | Hexyldecyllaurate | 2,00 |
| | Tocopherylacetat | 0,20 |
| | Coffein | 0,30 |
| | Biotin | 0,02 |
| | Cyclomethicon | 5,00 |
| | Dimethicon | 0,50 |
| | Octylmethoxycinnamat | 1,50 |
| | Panthenol | 2,00 |
| | 1,2 Propylenglycol | 2,00 |
| | Acrylate/C10-30 Alkylacrylat-Crosspolymer | 0,10 |
| | AMP | 0,25 |
| | Haltbarmacher | n. B. |
| | Aqua | ad 100 |

| **Beispiel 6** | **- Gesichtsmaske -** | |
|---|---|---|
| | Glyceryl_Stearate | 10,00 |
| | Octyldodecanol | 8,00 |
| | Cetearyl_Ethylhexanoate | 8,00 |
| | Kaolin | 4,00 |
| | Caffeine | 0,50 |
| | Magnesium PCA | 1,00 |
| | Isopropylmyristate | 4,00 |
| | Sorbitol | 2,00 |
| | Panthenol | 3,00 |
| | Sodiumcetearylsulfate | 1,00 |
| | Parfum | 0,40 |
| | Haltbarmacher | n. B. |
| | Aqua | ad 100,00 |

| **Beispiel 7** | **- Reinigungsgel-** | |
|---|---|---|
| | Laurylglucosid | 10,00 |
| | Natrium-Laurethsulfat | 4,00 |
| | hydrolysiertes Gluten aus Weizen | 0,50 |
| | Panthenol | 0,50 |
| | Coffein | 0,20 |
| | Allantoin | 0,10 |
| | Natriumchlorid | 1,00 |
| | Parfum | 0,40 |
| | Haltbarmacher | n.B. |
| | Aqua | ad 100,00 |

| **Beispiel 8** | **- Duschgel -** | |
|---|---|---|
| | Natrium-Laurethsulfat | 5,00 |
| | Magnesium-Laurethsulfat | 2,00 |
| | Natrium-Olethsulfat | 3,00 |
| | Dinatrium-Laurethsulfosuccinat | 2,00 |
| | Kaliumcocoyl-hydrolysiertes Collagen | 2,00 |
| | Laurylglucosid | 3,00 |
| | hydrolysiertes Collagen | 1,00 |
| | Coffein | 0,40 |
| | Panthenol | 1,00 |
| | Glyceryllaurat | 1,00 |
| | Natriumchlorid | 3,00 |
| | Haltbarmacher | n.B. |
| | Aqua | ad 100,00 |

| **Beispiel 9** | **- Körperlotion -** | |
|---|---|---|
| | PEG-7 hydriertes Castoröl | 2,00 |
| | PEG-20 Glyceryllaurat | 1,00 |
| | Octylstearat | 4,00 |
| | Cetearylisonanoat | 4,00 |
| | Coffein | 0,15 |
| | Tocopherylacetat | 0,50 |
| | Carbomer | 0,40 |
| | Glycerin | 3,00 |
| | Haltbarmacher | n. B. |
| | Natriumhydroxid | 0,15 |
| | Aqua | ad 100,00 |

| **Beispiel 10** | **- Antitranspirant Spray -** | |
|---|---|---|
| | Glycerylstearat | 2,50 |
| | Ceteareth-20 | 2,50 |
| | Ceteareth-12 | 1,00 |
| | Coffein | 0,30 |
| | Cetearylalkohol | 1,00 |
| | Coco-Caprylat/Caprat | 5,00 |
| | Dicaprylylether | 5,00 |
| | Aluminumchlorohydrat | 8,00 |
| | Parfum | 0,80 |
| | Haltbarmacher | n.B. |
| | Aqua | ad 100,00 |

## Patentansprüche

1. Verwendung von Coffein zur Herstellung einer kosmetischen oder pharmazeutischen Formulierung zur Verbesserung des natürlichen Hautschutzes, wobei besagte Haut männliche Haut oder weibliche Haut mit erhöhtem Testosteronspiegel ist

2. Verwendung nach Anspruch 1, zur Behandlung und/oder Prophylaxe von Störungen, die mit einer mangelnden Regeneration der Epidermis verbunden sind.

3. Verwendung nach einem der zuvor genannten Ansprüchen wobei das Coffein in der Formulierung in einer Menge von 0,001 bis 30 Gew.-% vorliegt.

4. Verwendung nach Anspruch 3, wobei das Coffein in der Formulierung in einer Menge von 0,01 bis 2 Gew.-% vorliegt.

5. Verwendung nach irgendeinem der zuvor genannten Ansprüchen, wobei das Coffein in Kombination mit weiteren aktiven Komponenten verwendet wird, ausgewählt aus der Gruppe bestehend aus Vitaminen, Flavonoiden, Lichtschutzfiltem, feuchtigkeitsspendenden Wirkstoffen, Terpenoiden, Sacchariden, Pflanzenextrakten, Oligo- oder Polypeptiden und anderen sekundären Pflanzenbegleitstoffen.

6. Verwendung nach irgendeinem der vorstehenden Ansprüche, wobei die Formulierung in Form einer Emulsion, einer Liposomenzubereitung oder einer Nanospherenzubereitung vorliegt.

7. Verwendung nach Anspruch 6, wobei die Emulsion eine Emulsion vom Typ W/O, O/W oder W/O/W ist.

## Claims

1. Use of caffeine to prepare a cosmetic or pharmaceutical formulation for improving natural skin protection, wherein said skin is male skin or female skin with an elevated testosterone level.

2. Use according to Claim 1, for the treatment and/or prophylaxis of disorders which are associated with deficient regeneration of the epidermis.

3. Use according to either of the preceding Claims, wherein the caffeine is present in the formulation in a quantity of 0.001 to 30 % by weight.

4. Use according to Claim 3, wherein the caffeine is present in the formulation in a quantity of 0.01 to 2 % by weight.

5. Use according to any one of the preceding Claims, wherein the caffeine is used in combination with further active components selected from the group comprising vitamins, flavinoids, light protection filters, moisturising agents, terpenoids, saccharides, plant extracts, oligopeptides or polypeptides and other secondary plant accompanying substances.

6. Use according to any one of the preceding Claims, wherein the formulation is present in the form of an emulsion, a liposome preparation or a nanospherene preparation.

7. Use according to Claim 6, wherein the emulsion is an emulsion of w/o, o/w or w/o/w type.

## Revendications

1. Utilisation de la caféine pour la préparation d'une formulation cosmétique ou pharmaceutique pour l'amélioration de la protection naturelle de la peau, où ladite peau est une peau masculine où une peau féminine avec un taux de testostérone élevé.

2. Utilisation selon la revendication 1, pour le traitement et/ou la prophylaxie des troubles liés à une régénération défaillante de l'épiderme.

3. Utilisation selon l'une des revendications précédentes, où la caféine est présente dans la formulation en une quantité de 0,001 à 30% en poids.

4. Utilisation selon la revendication 3 où la caféine est présente dans la formulation dans une quantité de 0,01 à 2% en poids.

5. Utilisation selon l'une quelconque des revendications précédentes, où la caféine est utilisée en combinaison avec d'autres composants actifs, choisis dans le groupe constitué des vitamines, flavonoïdes, filtres protecteur de la lumière, principes actifs hydratants, terpénoïdes, saccharides, extraits de plantes, oligo- ou polypeptides et autres matières végétales secondaires.

6. Utilisation selon l'une quelconque des revendications précédentes, où la formulation se présente sous la forme d'une émulsion, d'une préparation de liposomes ou d'une préparation de nanosphères.

7. Utilisation selon la revendication 6, où l'émulsion est une émulsion du type eau dans huile, huile dans eau ou eau dans huile dans eau.
